# EUROPÄISCHE PATENTSCHRIFT

(11) **EP 1 589 095 B1**
(45) Veröffentlichungstag und Bekanntmachung des Hinweises auf die Patenterteilung: **12.07.2006**
(21) Anmeldenummer: 04009219.9
(22) Anmeldetag: 19.04.2004
(51) Int. Cl.: C12M 1/107, C05F 17/02

(54) **Vorrichtung zur Erzeugung und Verwertung von Biogas**
Device for producing and using biogas
Dispositif pour la production et l'utilisation de biogaz

(43) Veröffentlichungstag der Anmeldung: 26.10.2005
(73) Patentinhaber: Rückert, Claus, 91207 Lauf/Pegnitz (DE)
(72) Erfinder: Rückert, Claus, 91207 Lauf/Pegnitz (DE)
(74) Vertreter: Gassner, Wolfgang

(56) Entgegenhaltungen:
- WO-A-00/53542
- DE-A- 19 724 012
- DE-A- 19 746 636
- DE-B- 3 014 780
- DE-U- 20 016 591
- GB-A- 2 124 608

## Beschreibung

Die Erfindung betrifft eine Vorrichtung zur Erzeugung und Verwertung von Biogas nach dem Oberbegriff des Anspruchs 1.

Eine solche Vorrichtung ist beispielsweise aus dem Prospekt des Ingenieurbüros Claus Rückert, Biogasanlagen, 92259 Neukirchen bekannt. - Die bekannte Vorrichtung erfordert einen relativ hohen Herstellungsaufwand. Es müssen zwei separate Gebäude errichtet werden. Das eine Gebäude bildet die Hülle eines Biogasreaktors und umfasst ggf. auch einen Biogasspeicher. Ein zweites üblicherweise entfernt davon errichtetes Gebäude dient der Aufnahme eines Blockheizkraftwerks, welches mit dem im ersten Gebäude erzeugten Biogas betrieben wird. Die Notwendigkeit des Errichtens zweier Gebäude erfordert einen relativ hohen Platzbedarf. Das macht herkömmliche Vorrichtungen zur Erzeugung und Verwertung von Biogas teuer.

Aus der DE 199 58 142 A1 ist eine mobile Biogasanlage bekannt. Anstelle zweier separater Gebäude sind hier zwei separate Container vorgesehen. Im einen Container ist ein Fermenter untergebracht. Im anderen Container befindet sich ein Biogasspeicher sowie eine Vorrichtung zur Erzeugung von Energie. Zur Inbetriebnahme einer solchen mobilen Biogasanlage ist es erforderlich, zwischen den beiden Containern Strom- und Gasverbindungsleitungen zu installieren. Derartige Container können nebeneinander und auch teilweise übereinander aufgestellt werden. Nähere Angaben über die Anordnung der Container sind der DE 199 58 142 A1 nicht zu entnehmen.

Eine weitere mobile Biogasanlage ist aus der DE 197 44 653 A1 bekannt. Dabei sind in Modulbauweise aus vorgefertigten Bauteilen ein Biogasreaktor, ein Biogassammelsystem und ein Blockheizkraftwerk vorgesehen, die in einem einzigen Gehäuse integrierbar sind. Das Gehäuse kann mit samt der darin aufgenommenen Module transportiert werden. Die vorgeschlagene modulare Bauweise ermöglicht zwar eine Demontage der vorgeschlagenen Biogasanlage sowie deren Wiedererrichtung an einem anderen Ort. Die Herstellung einer modular aufgebauten Biogasanlage ist aber relativ aufwändig.

Die EP 1 069 174 A1 beschreibt einen Gaserzeuger zur Herstellung von Methangas. Durch Wärmerückgewinnung der von einem mit Biogas betriebenen Motor ausgestoßenen Abgase wird eine Dehydrierung der Biomasse ermöglicht. Der Motor ist außerhalb eines den Reaktor umgebenden Gebäudes angeordnet.

Die EP 1 088 885 A2 beschreibt einen weiteren Biogasreaktor. Über ein Leitungsnetz wird das damit hergestellte Gas zu einer Gasverwertungseinrichtung transportiert. Das Vorsehen und die Wartung eines Leitungsnetzes zum Transport von Biogas ist aufwändig.

Die DE 197 46 636 A offenbart ein teilweise in die Erde eingelassenes Gebäude, dass einen Raum aufweist, der als Fermenter fungiert, einen zweiten (Raum unterhalb des Dachs), der mit einer Folie abgeschlossen ist und als Gasspeicher dient und einen dritten Raum (21), in dem ein Blockheizkraftwerk angeordnet ist. Ferner weist das Gebäude eine für den pumpfähigen Anteil der Biomasse vorgesehene Heizeinrichtung auf (Heizschlange im Fermenter) und in einem vierten Raum eine Einrichtung zum An-/und Abtransport der Biomasse auf.

Aufgabe der Erfindung ist es, die Nachteile nach dem Stand der Technik zu beseitigen. Es soll insbesondere eine möglichst einfach und kostengünstig herstellbare Vorrichtung zur Erzeugung und Verwertung von Biogas angegeben werden. Nach einem weiteren Ziel der Erfindung soll die Vorrichtung möglichst kompakt aufgebaut sein. Die Vorrichtung soll ferner möglichst leicht mit Biomasse beschickbar und die beim Verfahren anfallenden Reststoffe sollen möglichst einfach entnehm- und abtransportierbar sein.

Diese Aufgabe wird durch die Merkmale des Anspruchs 1 gelöst. Zweckmäßige Ausgestaltungen ergeben sich aus den Merkmalen der Ansprüche 2 bis 16.

Nach Maßgabe der Erfindung ist vorgesehen, dass im Biogasreaktor ein Haspelrührwerk zum Rühren und Zerkleinem von fester Biomasse aufgenommen ist.

Zweckmäßigerweise ist der erste Raum Bestandteil eines Untergeschosses des Gebäudes und der zweite und dritte Raum sind Bestandteil eines Obergeschosses. Dabei können der zweite und/oder dritte Raum oberhalb des ersten Raums angeordnet sein. In dieser Ausgestaltung ist die Vorrichtung besonders kompakt ausgebildet.

Nach einer weiteren besonders vorteilhaften Ausgestaltung weist das Gebäude einen aus Beton, vorzugsweise Stahlbeton, hergestellten Baukörper auf. Zumindest eine Wand des Baukörpers kann eine Stützwand zum Abstützen einer bis zum Niveau des Obergeschosses reichenden Geländeauffüllung bilden. Eine solche Geländeauffüllung bis zum Niveau des Obergeschosses ermöglicht ein einfaches Anfahren von Fahrzeugen. Der Baukörper kann des Weiteren so angeordnet sein, dass an einer beispielsweise der Stützwand gegenüberliegenden Wand das Geländeniveau etwa dem Niveau des Untergeschosses entspricht. Das erleichtert das Anfahren von Fahrzeugen zum Beschicken des im Untergeschoss vorgesehenen Biogasreaktors mit Biomasse sowie zur Aufnahme von aus dem Biogasreaktor entnommenen Reststoffen.

Vorteilhafterweise ist der Biogasspeicher ein im einem zweiten Raum aufgenommener Foliengasspeicher. Im Foliengasspeicher kann eine Einrichtung zur Reinigung des Biogases aufgenommen sein. Die Einrichtung kann eine Gaswascheinrichtung, insbesondere zur Entfernung löslicher schwefel- und ammoniakhaltiger Verbindungen sein. Abgesehen davon kann die Einrichtung Aufwuchsflächen für Mikroorganismen zur Entfernung von im Biogas enthaltene Schadstoffen, insbesondere Schwefelverbindungen, umfassen. Ein Biogasspeicher mit den vorgenannten Merkmalen liefert ein relativ sauberes, im Wesentlichen aus Methan und Kohlendioxid bestehendes Biogas, welches sogleich, beispielsweise durch Verbrennung in einem Blockheizkraftwerk, einem Motor und dgl. in Energie, insbesondere elektrische Energie, umgewandelt werden kann.

Nach einer weiteren Ausgestaltung ist vorgesehen, dass eine den zweiten vom dritten Raum trennende Wand als Feuerschutzwand ausgebildet ist. Damit wird sichergestellt, dass es bei einer Funken- oder Flammenbildung im dritten Raum es zu keiner Explosion der Vorrichtung kommen kann.

In weiterer vorteilhafter Ausgestaltung ist eine Heizeinrichtung zum Erwärmen eines pumpfähigen Bestandteils der Biomasse vorgesehen. Die Heizeinrichtung kann einen Wärmetauscher und/oder eine Zerkleinerungseinrichtung aufweisen. Sie ist zweckmäßigerweise außerhalb des Gebäudes angeordnet. Das macht die Heizeinrichtung leicht zugänglich. Im Falle einer Reparatur oder Wartung muss der Biogasreaktor nicht entleert werden.

Nach einer weiteren Ausgestaltung kann im Untergeschoss neben dem ersten Raum ein vierter Raum vorgesehen sein, in dem eine Pumpeinrichtung zum Abziehen von vergorenen Reststoffen vorgesehen ist. Im Obergeschoss oberhalb des vierten Raums kann ein fünfter Raum vorgesehen sein, in dem eine Einrichtung zur Trennung der abgezogenen Reststoffe vorgesehen ist. Mittels einer solchen Einrichtung zur Trennung, die auch als "Separator" bezeichnet wird, werden die aus dem Biogasreaktor abgezogenen Reststoffe getrennt in eine im Wesentlichen feste und eine im Wesentlichen flüssige Phase. Die flüssige Phase wird zweckmäßigerweise rezirkuliert, d.h. in den Biogasreaktor zuzurückgeführt. Damit kann eine besonders effektive Vergärung und infolgedessen eine besonders hohe Ausbeute an Biogas erreicht werden.

Nach einer besonders vorteilhaften Ausgestaltung kann der Biogasreaktor so ausgebildet sein, dass er zur Vergärung ausschließlich mit fester Biomasse, beispielsweise mit Mais und dgl., beschickt wird. Es handelt sich in diesem Fall um einen Biogasreaktor, mit dem das Verfahren der "Feststoffvergärung" bzw. "Trockenfermentation" durchführbar ist. Die zur Vergärung bei den vorgenannten Verfahren erforderliche Flüssigkeit wird hier durch Rezirkulation von im Separator gewonnener flüssiger Phase in Gang gehalten.

Zweckmäßigerweise ist eine Einrichtung zum Zuführen von Biomasse zum Bioreaktor außerhalb des Gebäudes vorgesehen. Das erleichtert deren Beschicken. Außerdem ist sie im Falle einer Wartung oder Reparatur besonders leicht zugänglich. Nach einer besonders vorteilhaften Ausgestaltung bildet die Einrichtung zum Zuführen von Biomasse ein, beispielsweise in einem Container aufgenommenes, Modul, das nötigenfalls ausgetauscht werden kann.

Nachfolgend werden Ausführungsbeispiele der Erfindung anhand der Zeichnungen näher erläutert. Es zeigen:
- Fig. 1: einen Grundriss des Untergeschosses eines ersten Gebäudes,
- Fig. 2: einen Grundriss des Obergeschosses des ersten Gebäudes,
- Fig. 3: eine erste Querschnittsansicht gemäß Fig. 1 und 2, und
- Fig. 4: eine zweite Querschnittsansicht gemäß Fig. 1 und 2,
- Fig. 5: einen Grundriss des Untergeschosses eines zweiten Gebäudes,
- Fig. 6: einen Grundriss des Obergeschosses des zweiten Gebäudes,
- Fig. 7: eine erste Querschnittsansicht gemäß Fig. 5 und 6 und
- Fig. 8: eine zweite Querschnittsansicht gemäß Fig. 5 und 6.

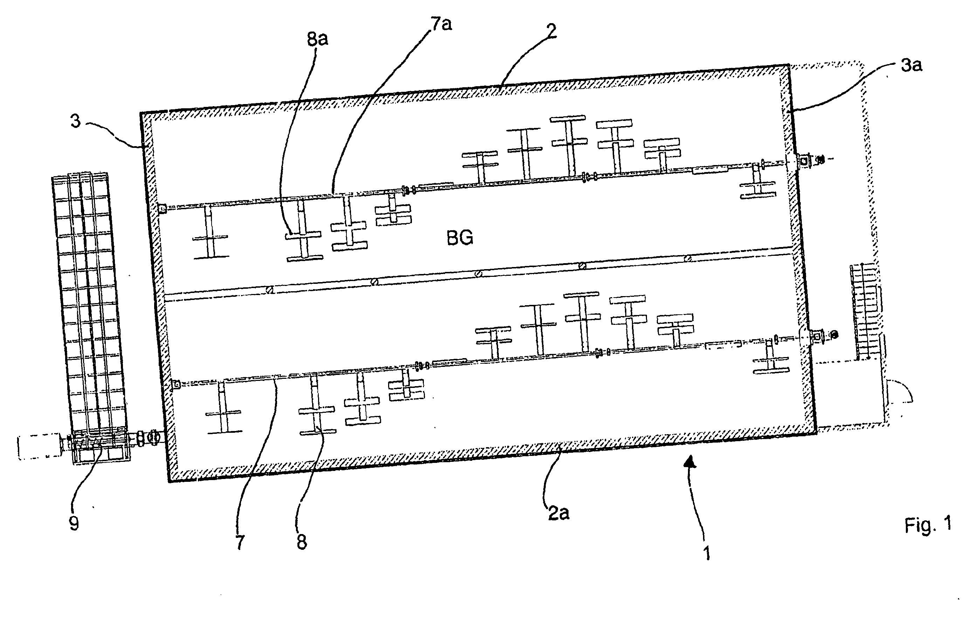

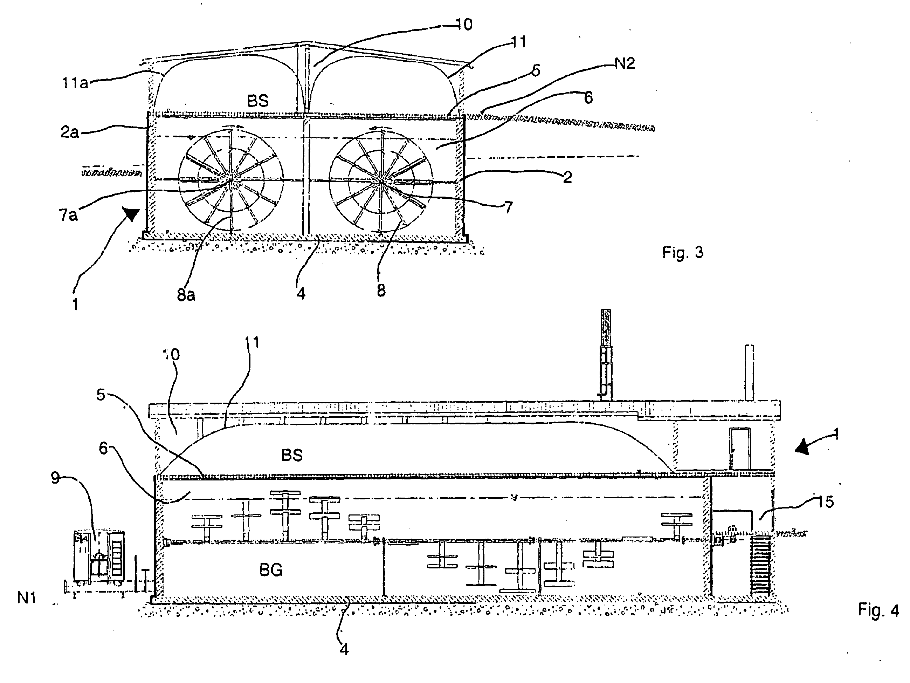

In den Fig. 1 bis 4 ist ein erstes Ausführungsbeispiel einer erfindungsgemäßen Vorrichtung gezeigt. Ein allgemein mit dem Bezugszeichen 1 bezeichnetes erstes Gebäude ist von zwei langen 2, 2a und zwei kurzen Außenwänden 3, 3a umgeben. Die langen 2, 2a und die kurzen Außenwände 3, 3a bilden zusammen mit der aus den Fig. 3 und 4 ersichtlichen Bodenplatte 4 sowie mit der Decke 5 einen ersten Raum 6. Die Außenwände, 2, 2a, 3, 3a, die Bodenplatte 4 sowie die Decke 5 bilden einen Baukörper, der aus Beton, insbesondere Stahlbeton, hergestellt sein kann. Der erste Raum 6 bildet zugleich die Hülle eines Biogasreaktors BG. Zwischen den beiden kurzen Außenwänden 3, 3a sind um im Wesentlichen horizontale Achsen 7, 7a drehbare Haspelrührwerke 8, 8a aufgenommen. Die vorgeschlagenen Haspelrührwerke 8, 8a ermöglichen eine besonders energiesparende Durchmischung der im Biogasreaktor BG aufgenommenen Biomasse. Es wird eine effektive Umsetzung der Rührenergie erreicht.

Unerwünschte Sink- und Schwimmschichten werden zuverlässig vermieden. Mit dem Bezugszeichen 9 ist eine Einrichtung zum Beschicken des Biogasreaktors BG mit einer (hier nicht gezeigten) Biomasse bezeichnet.

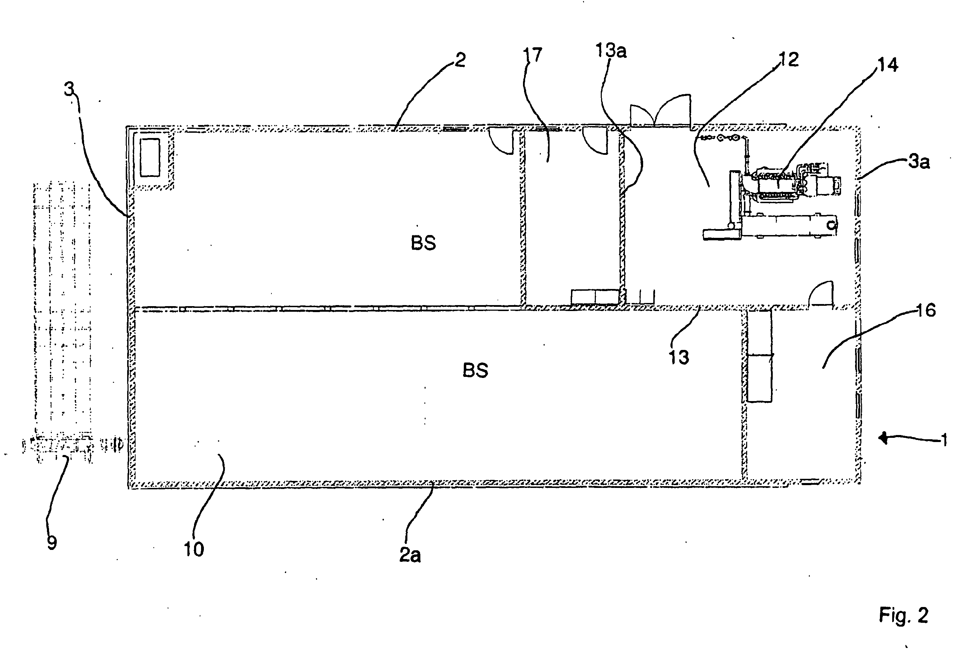

Bei dem in Fig. 2 gezeigten Obergeschoss begrenzen jeweils abschnittsweise die langen Außenwände 2, 2a und die kurze Außenwand 3 einen zweiten Raum 10, in dem ein Biogasspeicher BS untergebracht ist. Der Biogasspeicher BS umfasst insbesondere zwei Foliengasspeicher 11, 11a. In den Foliengasspeichern 11, 11a können (hier nicht gezeigte) Aufwuchsflächen zur Aufnahme von Mikroorganismen sowie eine Gaswascheinrichtung vorgesehen sein. Derartige Einrichtungen dienen der Entfernung von Schadstoffen, insbesondere schwefelhaltigen Verbindungen, aus dem Biogas.

Ein im Obergeschoss jeweils abschnittsweise von der kurzen Außenwand 3a und der langen Außenwand 2 begrenzter dritter Raum 12 ist gebäudeinnenseitig über Feuerschutzwände 13, 13a begrenzt. Im dritten Raum 12 ist ein Blockheizkraftwerk 14 aufgenommen. Anstelle des Blockheizkraftwerks 14 können selbstverständlich auch andere geeignete Einrichtungen zur Erzeugung von Energie, insbesondere elektrische Energie, vorgesehen sein. Im dritten Raum 12 kann ferner eine dem Blockheizkraftwerk 14 vorgeschaltete Einrichtung zur Erhöhung des Biogasdrucks vorgesehen sein. Ferner kann sich vom dritten Raum 12 durch ein Dach der Vorrichtung hindurch eine Einrichtung zum Abfackeln von Biogas, eine so genannte "Gasfackel" erstrecken. Angrenzend an den zweiten und/oder dritten Raum ist ein fünfter Raum 16 vorgesehen, in dem ein (hier nicht gezeigter) Separator zur Trennung von aus dem Biogasreaktor BG entfernten Reststoffen aufgenommen ist. Unterhalb des fünften Raums 16 kann - wie aus Fig. 4 ersichtlich ist - ein vierter Raum 15 vorgesehen sein, der beispielsweise zur Aufnahme von Pumpen dienen kann. Mittels der Pumpen ist es möglich, wahlweise vergorene Biomasse bzw. Reststoffe aus dem Biogasreaktor BG abzuziehen. Die Pumpen können automatisch gesteuert oder auch geregelt werden. Im Obergeschoss kann angrenzend an den zweiten Raum 10 ein sechster Raum 17 vorgesehen sein, der zur Aufnahme von Schaltanlagen (hier nicht gezeigt) dienen kann.

Wie insbesondere aus den Fig. 3 und 4 ersichtlich ist, befindet sich ein erstes Geländeniveau N1 etwa auf dem Niveau des Untergeschosses. Ein zweites Geländeniveau N2 ist durch eine Aufschüttung gegen die als Stützwand wirkende Längswand 2 gebildet. Das zweite Geländeniveau N2 entspricht etwa dem Niveau des Obergeschosses. Sämtliche Wände 2, 2a, 3, 3a, 13, 13a sowie die Bodenplatte 4 und die Decke 5 sind zweckmäßigerweise aus Stahlbeton hergestellt.

Die Anordnung der Räume 6, 10, 12, 15, 16, 17 ist so gewählt, dass die Länge von Verbindungsleitungen zum Stofftransport minimal ist. So ist der vierte Raum 15 zur Aufnahme von Pumpen unmittelbar benachbart dem ersten Raum 6 angeordnet. Das erleichtert den Austrag und Transport von Reststoffen aus dem ersten Raum 6 und deren Beförderung in den darüberliegenden fünften Raum 16, in dem ein Separator zur Trennung der Reststoffe aufgenommen ist. Das im Biogasreaktor BG gebildete Biogas kann ohne das Vorsehen besonders langer Leitungen ohne weiteres in den darüber befindlichen Biogasspeicher BS überführt werden. Desgleichen kann das im Biogasspeicher BS gespeicherte Biogas ohne das Vorsehen langer Leitungen sofort dem im dritten Raum 12 aufgenommenen Blockheizkraftwerk 14 zugeführt werden.

Die auf dem ersten Niveau N1 vorgesehene Einrichtung 9 zum Beschicken des Biogasreaktors BG kann als Modul in Form eines Containers ausgeführt sein. Sie ist zweckmäßigerweise außerhalb des ersten Gebäudes 1 angeordnet. Das erleichtert deren Zugänglichkeit sowie die Beschickung des Biogasreaktors BG. Ein zweites Geländeniveau N2 ist so ausgebildet, dass damit ohne weiteres im Obergeschoss anfallende Reststoffe, insbesondere mittels des Separators abgetrennte Feststoffe, abgefahren werden können.

Beispielsweise an der kurzen Außenwand 3 kann ferner ein (hier nicht gezeigter) Doppelrohrwärmetauscher zur Vorwärmung pumpfähiger Biomasse vorgesehen sein. Mittels des Doppelrohrwärmetauschers kann ein pumpfähiger Bestandteil der Biomasse im Kreislauf durch den Biogasreaktor BG gepumpt und ständig auf einer gewünschten vorgegebenen Temperatur gehalten werden. Der Doppelrohrwärmetauscher kann einen Zerkleinerer umfassen, mit dem besonders energiesparend im pumpfähigen Bestandteil der Biogas enthaltene fasrige Bestandteile aufgelöst bzw. desintegriert werden können. Mit einem solchen Zerkleinern der fasrigen Bestandteile kann weiter die Ausbeute an Biogas erhöht werden.

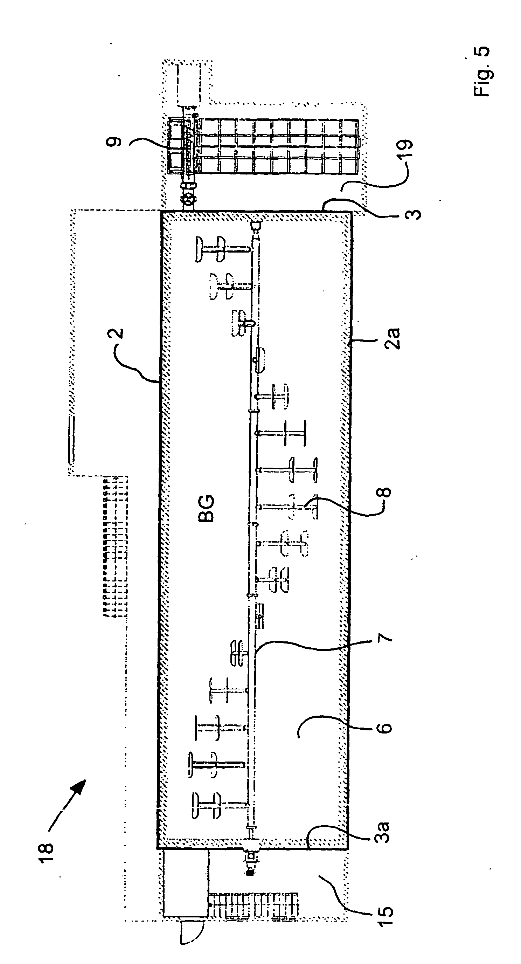

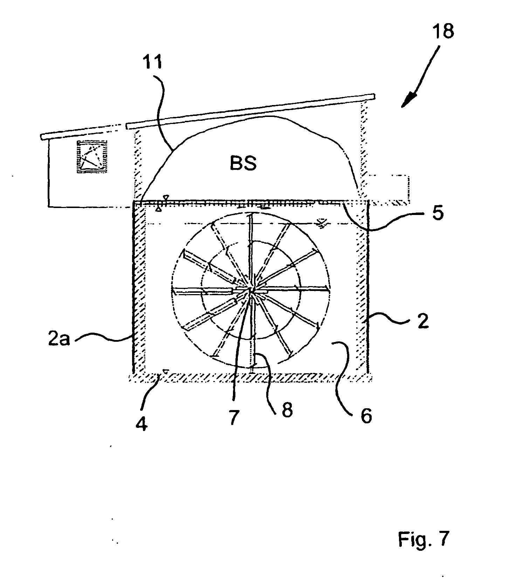

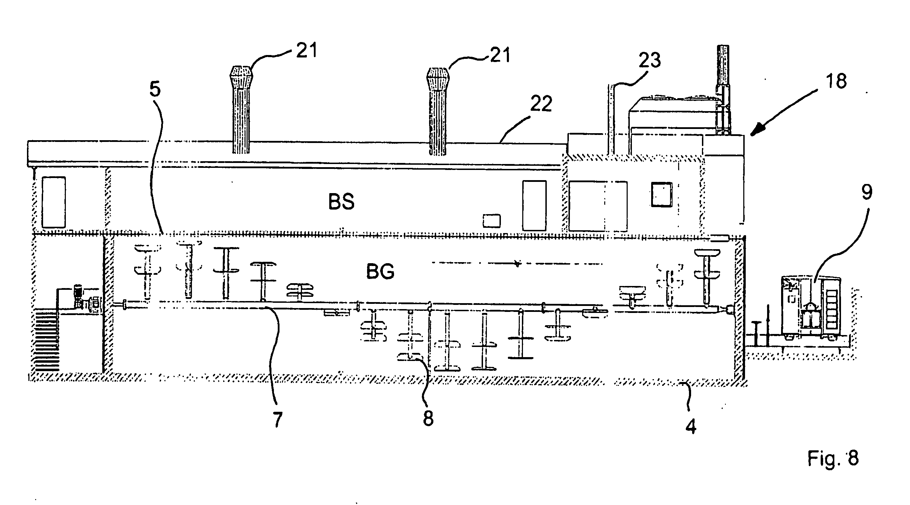

Die Fig. 5 bis 8 zeigen ein zweites Ausführungsbeispiel einer erfindungsgemäßen Biogasanlage. Ein allgemein mit dem Bezugszeichen 18 bezeichnetes zweites Gebäude weist im Untergeschoss wiederum einen ersten Raum 6, der von zwei langen 2, 2a und zwei kurzen Außenwänden 3, 3a umgeben ist. Der erste Raum 6 bildet wiederum die Hülle des Biogasreaktors BG. Darin ist ein um die horizontale Achse 7 drehbares Haspelrührwerk 8 aufgenommen. Die Einrichtung 9 zum Beschicken des Biogasreaktors BG ist hier in einem an den ersten Raum 6 angebauten siebten Raum 19 untergebracht. Gegenüberliegend dem siebten Raum schließt sich an den ersten Raum 6 der vierte Raum 15 an, in dem (hier nicht gezeigte) Pumpen zum aktiven Austragen von vergorenen Reststoffen aus dem Biogasreaktor BG untergebracht sind.

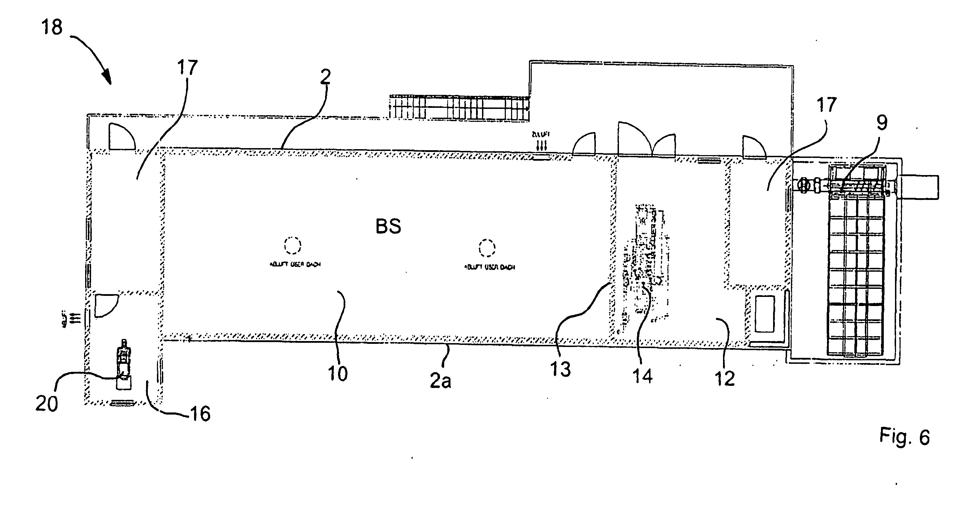

Wie insbesondere aus Fig. 6 ersichtlich ist, befindet sich oberhalb des vierten Raums wiederum der fünfte Raum 16, in dem ein mit dem Bezugszeichen 20 bezeichneter Separator aufgenommen ist. Die Feuerschutzwand 13 trennt den im zweiten Raum 10 aufgenommenen Biogasspeicher BS vom dritten Raum 12, in dem das Blockheizkraftwerk 14 sich befindet. Ferner sind im in Fig. 6 gezeigten Obergeschoss zwei sechste Räume 17 vorgesehen, die beispielsweise als Montage- oder Schaltraum nutzbar sind.

Der zweite Raum 10 ist zur Belüftung mit Abluftkaminen 21 versehen. Vom dritten Raum 12 erstreckt sich durch ein Dach 22 eine Gasfackel 23.

### Bezugszeichenliste

- 1: erstes Gebäude
- 2, 2a: lange Außenwände
- 3, 3a: kurze Außenwände
- 4: Bodenplatte
- 5: Decke
- 6: erster Raum
- 7, 7a: Achse
- 8, 8a: Haspelrührwerk
- 9: Einrichtung zum Beschicken
- 10: zweiter Raum
- 11, 11a: Foliengasspeicher
- 12: dritter Raum
- 13, 13a: Feuerschutzwand
- 14: Blockheizkraftwerk
- 15: vierter Raum
- 16: fünfter Raum
- 17: sechster Raum
- 18: zweites Gebäude
- 19: siebter Raum
- 20: Separator
- 21: Abluftkammer
- 22: Dach
- 23: Gasfackel

- BG: Biogasreaktor
- BS: Biogasspeicher
- N1: erstes Geländeniveau
- N2: zweiter Geländeniveau

## Patentansprüche

1. Vorrichtung zur Erzeugung und Verwertung von Biogas mit einem mehrere Räume (6, 10, 12, 15, 16, 17) aufweisenden Gebäude (1), wobei ein erster Raum (6) die Hülle eines Biogasreaktors (BG) bildet und ein zweiter Raum (10) einen Biogasspeicher (BS) umgibt, und wobei das Gebäude (1) zumindest einen dritten Raum (12) aufweist, in dem eine Einrichtung (14) zur Umwandlung von Biogas in elektrische Energie aufgenommen ist,
**dadurch gekennzeichnet, dass**
im Biogasreaktor (BG) ein Haspelrührwerk (8, 8a) zum Rühren und Zerkleinern von fester Biomasse aufgenommen ist.

2. Vorrichtung nach Anspruch 1, wobei der erste Raum (6) Bestandteil einer Untergeschosses des Gebäudes (1) ist und der zweite (10) und dritte Raum (12) Bestandteil eines Obergeschosses sind.

3. Vorrichtung nach einem der vorhergehenden Ansprüche, wobei der zweite (10) und/oder dritte Raum (12) oberhalb des ersten Raums (6) angeordnet sind.

4. Vorrichtung nach einem der vorhergehenden Ansprüche, wobei das Gebäude (1) einen aus Beton, vorzugsweise Stahlbeton, hergestellten Baukörper aufweist.

5. Vorrichtung nach einem der vorhergehenden Ansprüche, wobei zumindest eine Wand (2) des Baukörpers eine Stützwand zum Abstützen einer bis zum Niveau (N2) des Oberschosses reichenden Geländeauffüllung bildet.

6. Vorrichtung nach einem der vorhergehenden Ansprüche, wobei der Biogasspeicher (BS) ein im zweiten Raum (10) aufgenommener Foliengasspeicher (11, 11a) ist.

7. Vorrichtung nach einem der vorhergehenden Ansprüche, wobei im Foliengasspeicher (11, 11a) eine Einrichtung zur Reinigung des Biogases aufgenommen ist.

8. Vorrichtung nach einem der vorhergehenden Ansprüche, wobei die Einrichtung zur Reinigung eine Gaswascheinrichtung, insbesondere zur Entfernung löslicher schwefel- und ammoniakhaltiger Verbindungen, ist.

9. Vorrichtung nach einem der vorhergehenden Ansprüche, wobei die Einrichtung zur Reinigung Aufwuchsflächen für Mikroorganismen zur Entfernung von im Biogas enthaltenen Schadstoffen, insbesondere Schwefelverbindungen, umfasst.

10. Vorrichtung nach einem der vorhergehenden Ansprüche, wobei eine den zweiten (10) vom dritten Raum (12) trennende Wand (13, 13a) als Feuerschutzwand ausgebildet ist.

11. Vorrichtung nach einem der vorhergehenden Ansprüche, wobei eine Heizeinrichtung zum Erwärmen eines pumpfähigen Bestandteils des Biomasse vorgesehen ist.

12. Vorrichtung nach einem der vorhergehenden Ansprüche, wobei die Heizeinrichtung einen Wärmetauscher und/oder eine Zerkleinerungseinrichtung aufweist.

13. Vorrichtung nach einem der vorhergehenden Ansprüche, wobei die Heizeinrichtung außerhalb des Gebäudes (1) angeordnet ist.

14. Vorrichtung nach einem der vorhergehenden Ansprüche, wobei im Untergeschoss neben dem ersten Raum (6) eine vierter Raum (15) vorgesehen ist, in dem eine Pumpeinrichtung zum Abziehen von im Biogasreaktor (BG) aufgenommenen Reststoffen vorgesehen ist.

15. Vorrichtung nach einem der vorhergehenden Ansprüche, wobei im Obergeschoss oberhalb des vierten Raums (15) ein fünfter Raum (16) vorgesehen ist, in dem eine Einrichtung zur Trennung der aus dem Biogasreaktor (BG) abgezogenen Reststoffe vorgesehen ist.

16. Vorrichtung nach einem der vorhergehenden Ansprüche, wobei eine Einrichtung (9) zum Zuführen von Biomasse zum Bioreaktor (BG) außerhalb des Gebäudes (1) vorgesehen ist.

## Claims

1. Device for producing and using biogas with a building (1) having several rooms (6, 10, 12, 15, 16, 17), wherein a first room (6) forms the shell of a biogas reactor (BG) and a second room (10) surrounds a biogas reservoir (BS), and wherein the building (1) has at least a third room (12) containing a unit (14) for converting biogas into electrical energy
**characterized in that**
the biogas reactor (BG) contains a reel agitator (8, 8a) for stirring and breaking up solid biomass.

2. Device as defined in claim 1, wherein the first room (6) is part of a basement of the building (1) and the second (10) and third room (12) are part of an upper floor.

3. Device as defined in one of the preceding claims, wherein the second (10) and/or third room (12) are located above the first room (6).

4. Device as defined in one of the preceding claims, wherein the building (1) has a construction body made of concrete, preferably steel-reinforced concrete.

5. Device as defined in one of the preceding claims, wherein at least one wall (2) of the construction body forms a support wall to support a land fill which reaches up to the level (N2) of the upper floor.

6. Device as defined in one of the preceding claims, wherein the biogas reservoir (BS) is a membrane gas reservoir (11, 11a) located in the second room (10).

7. Device as defined in one of the preceding claims, wherein the membrane gas reservoir (11, 11a) contains a device for purifying the biogas.

8. Device as defined in one of the preceding claims, wherein the unit is for purifying a gas washing unit, in particular for removing soluble compounds containing sulfur and ammoniac.

9. Device as defined in one of the preceding claims, wherein the unit for purifying includes periphyton surfaces for microorganisms for the removal of pollutants, in particular sulfur compounds, contained in the biogas.

10. Device as defined in one of the preceding claims, wherein a wall (13, 13a) separating the second room (10) from the third room (12) is designed as a fire wall.

11. Device as defined in one of the preceding claims, wherein a heating unit for heating a pumpable part of the biomass is provided.

12. Device as defined in one of the preceding claims, wherein the heating unit has a heat exchanger and/or a chopping unit.

13. Device as defined in one of the preceding claims, wherein the heating unit is located outside the building (1).

14. Device as defined in one of the preceding claims, wherein a fourth room (15) is located in the basement next to the first room (6) in which fourth room a pumping unit is provided to pump off the residues collected in the biogas reactor (BG).

15. Device as defined in one of the preceding claims, wherein a fifth room (16) is located on the upper floor above the fourth room (15), in which fifth room a unit is provided for the separation of the residues which were pumped out of the biogas reactor (BG).

16. Device as defined in one of the preceding claims, wherein a unit (9) is provided to feed the biomass to the bioreactor (BG) outside the building (1).

## Revendications

1. Dispositif pour la production et la valorisation du biogaz, comprenant un bâtiment (1) comptant plusieurs locaux (6,10, 12, 15, 16, 17), un premier local (6) formant l'enceinte d'un réacteur du biogaz (BG) et un deuxième local (10) entourant un réservoir de biogaz (BS), et le bâtiment (1) présentant au moins un troisième local (12) qui abrite un dispositif (14) pour transformer le biogaz en énergie électrique, **caractérisée en ce que** le réacteur du biogaz (BG) renferme un mélangeur à tourniquet (8, 8a) destiné à mélanger et à fragmenter la biomasse solide.

2. Dispositif selon la revendication 1, le premier local (6) faisant partie d'un étage inférieur du bâtiment (1) et les deuxième et troisième locaux (10, 12) faisant partie d'un étage supérieur du bâtiment.

3. Dispositif selon l'une des revendications précédentes, le deuxième (10) et/ou le troisième local (12) étant situé au-dessus du premier local (6).

4. Dispositif selon l'une des revendications précédentes, le bâtiment (1) présentant une structure en béton, de préférence en béton armé.

5. Dispositif selon l'une des revendications précédentes, au moins un mur (2) de la structure constituant un mur porteur destiné à supporter un remblai de terre s'étendant jusqu'au niveau (N2) de l'étage supérieur du bâtiment.

6. Dispositif selon l'une des revendications précédentes, le réservoir de biogaz (BS) étant un réservoir de gaz souple (11, 11a) installé dans le second local (10).

7. Dispositif selon l'une des revendications précédentes, un dispositif pour purifier le biogaz étant placé dans le réservoir de gaz souple (11, 11a).

8. Dispositif selon l'une des revendications précédentes, le dispositif de purification étant un dispositif de lavage de gaz, notamment afin d'en retirer les composés solubles contenant du soufre et de l'ammoniac.

9. Dispositif selon l'une des revendications précédentes, le dispositif de purification comprenant des surfaces destinées à faire proliférer des micro-organismes, afin d'éliminer des matières toxiques contenues dans le biogaz, notamment les composés soufrés.

10. Dispositif selon l'une des revendications précédentes, un mur (13, 13a) séparant le deuxième local (10) du troisième local (12), en tant que mur de protection contre l'incendie.

11. Dispositif selon l'une des revendications précédentes, un dispositif de chauffage étant prévu pour chauffer un composant liquide de la biomasse.

12. Dispositif selon l'une des revendications précédentes, le dispositif de chauffage comportant un échangeur thermique et/ou un dispositif de fragmentation.

13. Dispositif selon l'une des revendications précédentes, le dispositif de chauffage étant placé à l'extérieur du bâtiment (1).

14. Dispositif selon l'une des revendications précédentes, un quatrième local (15) étant prévu à l'étage inférieur, à côté du premier local (6), dans lequel est prévu un dispositif de pompage pour extraire les matières résiduaires contenues dans le réacteur du biogaz (BG).

15. Dispositif selon l'une des revendications précédentes, un cinquième local (16) étant prévu à l'étage supérieur, au-dessus du quatrième local (15), dans lequel est prévu un dispositif destiné à séparer les matières résiduaires extraites du réacteur du biogaz (BG).

16. Dispositif selon l'une des revendications précédentes, un dispositif (9) destiné à alimenter le réacteur du biogaz (BG) en biomasse est prévu à l'extérieur du bâtiment (1).
